Europäisches Patentamt

European Patent Office

Office européen des brevets

(19)

(11) Veröffentlichungsnummer: **0 114 416**
**A1**

(12) **EUROPÄISCHE PATENTANMELDUNG**

(21) Anmeldenummer: 83113257.6

(22) Anmeldetag: 31.12.83

(51) Int. Cl.³: **C 07 C 11/08**
C 07 C 2/30, C 08 F 210/02
//(C08F210/02, 210/08)

(30) Priorität: 15.01.83 DE 3301162

(43) Veröffentlichungstag der Anmeldung:
01.08.84 Patentblatt 84/31

(84) Benannte Vertragsstaaten:
BE DE FR GB NL

(71) Anmelder: EC ERDÖLCHEMIE GMBH
Postfach 75 20 02
D-5000 Köln 71(DE)

(72) Erfinder: Schleppinghoff, Bernhard, Dr.
Adolf-Kolping-Strasse 5
D-4047 Dormagen 1(DE)

(72) Erfinder: Herwig, Jens, Dr.
Auf dem Hügel 23
D-5000 Köln 41(DE)

(72) Erfinder: Scheef, Hans-Volker
Goethestrasse 69
D-4047 Dormagen 1(DE)

(74) Vertreter: Dill, Erwin, Dr. et al,
c/o BAYER AG Zentralbereich Patente Marken und
Lizenzen Bayerwerk
D-5090 Leverkusen 1(DE)

(54) Verfahren zur Herstellung von Buten-1.

(57) Buten-1 wird durch Dimerisierung von Ethylen in Gegenwart eines Mischkatalysators aus einem Titan- oder Zirkonsäurealkylester und einem Aluminiumorganyl bei erhöhter Temperatur in einem Lösungsmittel hergestellt, wobei man zwischen 90 und 200°C arbeitet und eine Menge von $1 \times 10^{-6}$ bis $5 \times 10^{-3}$ Mol der Titan- oder Zirkonkomponente und eine Menge von $1 \times 10^{-4}$ bis $3 \times 10^{-2}$ Mol der Aluminiumkomponente pro Mol Ethylen einsetzt. Das Buten-1 kann hierbei auch im Gemisch mit Ethylen erhalten werden. Dieses Gemisch kann direkt für die Herstellung von Ethylen/Buten-1-Copolymerisaten eingesetzt werden.

EP 0 114 416 A1

EC Erdölchemie GmbH                    Köln-Worringen

Ha/bc/c    14. 01. 85

# Verfahren zur Herstellung von Buten-1

Die vorliegende Erfindung betrifft ein Verfahren zur Herstellung von Buten-1 durch Dimerisierung von Ethylen in Gegenwart eines Mischkatalysators aus einem Titan- oder Zirkonsäurealkylester und einem Aluminiumorganyl. In besonderer Weise betrifft die vorliegende Erfindung ein Verfahren zur Herstellung eines Gemisches aus Ethylen und Buten-1, das direkt für die Herstellung von Ethylen/Buten-1-Copolymerisaten verwendet werden kann.

Buten-1 findet weitgehende Verwendung, beispielsweise für die Herstellung von Alkylaten, Dimerisaten, Co-Dimerisaten, höheren Oligomerisaten, Polymerisaten und Copolymerisaten. Insbesondere die Herstellung von Ethylen/Buten-1-Copolymerisaten (ebenso wie andere Ethylen/$\alpha$-Olefin-Copolymerisate als LLDPE = Linear Low Density Polyethylene bezeichnet) gewinnt zunehmend an Bedeutung in den Einsatzgebieten, die bisher vom Hochdruckpolyethylen mit niedriger Dichte (LDPE = Low Density Polyethylene) beherrscht wurden, da eine Reihe von Eigenschaften und die Herstellkosten von LLDPE Vorteile gegenüber denen des LDPE zeigen. Die Herstellung von LLDPE

EC 144 - Ausland

.erfordert jedoch im Gegensatz zu der des LDPE außer dem Ethylen noch ein 1-Olefin, wie beispielsweise das Buten-1.

Als Quelle für Buten-1 dienen bisher vor allem die $C_4$-Raffinate aus thermischen Naphtha-Krackern nach der Abtrennung von Butadien und i-Buten. Das Buten-1 wird aus den verbleibenden $C_4$-Strömen destillativ durch Superfraktionierung oder durch Extraktivdestillation gewonnen. Für den Fall, daß in Zukunft die Ethylenerzeugung in zunehmendem Maße durch das Kracken von preiswerterem Ethan erfolgen wird, stehen die o.g. $C_4$-Ströme in geringeren Mengen als heute zur Verfügung. Andererseits wird bei zunehmendem Vordringen von LLDPE durch Substitution des bisherigen LDPE der Bedarf an Buten-1 steigen. Durch diese Verschiebungen wird nunmehr die Herstellung von Buten-1 aus Ethylen interessant.

An eine derartige Herstellung von Buten-1 aus Ethylen werden jedoch zahlreiche Anforderungen gestellt. So muß diese Dimerisierung mit hoher Selektivität zu dem gewünschten $C_4$-Olefin laufen, ohne daß größere Anteile von höheren Oligomeren oder gar Polymeren des Ethylens entstehen. Ferner sollte die Selektivität zum Buten-1 möglichst hoch sein, so daß wenig oder kein Buten-2 gebildet wird. Außerdem sollten die Kosten für den Katalysator möglichst niedrig gehalten werden, da schon das Ethylen als Ausgangsstoff einen hohen Wert besitzt.

Es sind bereits Verfahren zur Dimerisierung bzw. Oligomerisierung von Ethylen bekannt. Nach Angew. Chem. 68, 306 (1956) gelingt die Dimerisierung des Ethylens bei 70-90°C in Gegenwart von 10 g Titansäuretetraethylester

EC 144

und 20 g Triethylaluminium, wobei neben Hexen, Octen und Polyethylen in 2 Stunden 45 g n-Buten entsteht, das zu 2/3 aus Buten-1 und zu 1/3 aus Buten-2 besteht.

Nach J. Molecul. Catal. 6, 79-97 (1979) lassen sich beispielsweise mit $\eta$-Cycloocten-4-yl-1-(2,4-pentandionato)-nickel-Verbindungen, die gegebenenfalls fluorsubstituiert sein können, Butene aus Ethylen erzeugen. Außer dem gewünschten Dimeren bilden sich jedoch immer in merklichen Mengen höhere Oligomere, so daß die Butenselektivität des Katalysators unbefriedigend bleibt.

Weiterhin sind spezielle Komplexe von Chrom und Tantal als Dimerisierungskatalysatoren für Ethylen erwähnt (US 3 567 731 und J. Am. Chem. Soc. 100, 1315 (1978)). Alle zuvor erwähnten homogenen Katalysatoren sind jedoch nicht in der Lage, alle Anforderungen, die oben aufgezählt wurden, zu erfüllen. Es besteht daher nach wie vor der Wunsch, Buten-1 mit höherer Selektivität aus Ethylen zu gewinnen. Darüber hinaus besteht der Wunsch, ein Monomerengemisch aus Ethylen und Buten-1 aus Ethylen herzustellen. Insbesondere für die Herstellung dieses Gemisches sollte der Katalysator mit sehr hoher Selektivität zum Buten-1 arbeiten, so daß eine anschließende Produktaufarbeitung über Destillation oder Extraktivdestillation entfallen kann und das Ethylen/Buten-1-Gemisch zum direkten Einsatz in die Copolymerisation zum LLDPE geeignet ist.

Es wurde nun ein Verfahren zur Herstellung von Buten-1 durch Dimerisierung von Ethylen in Gegenwart eines Mischkatalysators aus einem Titan- oder Zirkonsäuretetraalkylester und einem Aluminiumorganyl bei erhöhter Tem-

EC 144

peratur in einem Lösungsmittel gefunden, das dadurch gekennzeichnet ist, daß man zwischen 90 und 200°C arbeitet und $1x10^{-6}$ bis $5x10^{-3}$ Mol der Titan- oder Zirkonkomponente und $1x10^{-4}$ bis $3x10^{-2}$ Mol der Aluminiumkomponente pro Mol Ethylen einsetzt.

Als erfindungsgemäß einsetzbare Titansäurealkylester und Zirkonsäurealkylester seien beispielsweise genannt: Tetramethoxy-titan, Tetraethoxy-titan, Tetrapropoxy-titan, Tetraisopropoxy-titan, Tetra-n-butoxy-titan, Tetrai-butoxy-titan oder gemischte Titansäurealkylester, wie Diethoxy-dipropoxy-titan, Dipropoxy-dimethoxy-titan oder Tri-n-butoxy-propoxy-titan oder halogenhaltige Titansäurealkylester, wie Dipropoxy-titan-dichlorid, Triethoxy-titanchlorid und ähnliche sowie die analogen Verbindungen des Zirkons.

Als erfindungsgemäß einsetzbare Aluminiumorganyle kommen beispielsweise Aluminiumtrialkyle, Alkylaluminiumhalogenide oder Aluminiumalkoxy-Verbindungen in Betracht. Vorzugsweise werden Aluminiumtrialkyle, wie Tri-i-butylaluminium, Isoprenylaluminium oder Triethylaluminium eingesetzt.

Die Titan- oder Zirkonkomponente wird in einer Menge von $1x10^{-6}$ bis $5x10^{-3}$ Mol, bevorzugt $1x10^{-5}$ bis $1x10^{-3}$ Mol pro Mol Ethylen eingesetzt. Die Aluminiumkomponente wird in einer Menge von $1x10^{-4}$ bis $3x10^{-2}$ Mol, bevorzugt $5x10^{-4}$ bis $1x10^{-2}$ Mol pro Mol Ethylen eingesetzt. Das Verhältnis der Titan- oder Zirkonkomponente einerseits und der Aluminiumkomponente andererseits zueinander ist

EC 144

grundsätzlich beliebig. Vielfach werden günstige Ergebnisse erzielt, wenn die Aluminiumkomponente in einer größeren Menge als die Titan- oder Zirkonkomponente im Reaktionsgemisch vorhanden ist. Das erfindungsgemäße Verfahren wird bei einer Temperatur zwischen 90 und 200°C, bevorzugt bei 92 bis 150°C, besonders bevorzugt bei 95 bis 130°C durchgeführt. Hierbei wird bei einem Gesamtdruck des Reaktionssystems von 5 bis 50 bar, bevorzugt 8 bis 35 bar, gearbeitet.

Die Umsetzung im erfindungsgemäßen Verfahren erfolgt in der flüssigen Phase eines gesättigten oder aromatischen Kohlenwasserstoffs, der mindestens 5 C-Atome haben sollte. Beispiele für geeignete Reaktionsmedien sind Pentan, Heptan, Isooctan, Isododecan, Benzol und Toluol.

Das Reaktionsgemisch kann beispielsweise destillativ aufgearbeitet werden, wobei das anfallende Ethylen in die Reaktion zurückgeführt wird. Hierbei wird ein Buten-1 mit einer Reinheit von etwa 99 Gew.-% erhalten.

In bevorzugter Weise wird jedoch die Reaktion des erfindungsgemäßen Verfahrens so gelenkt, daß ein Gemisch aus Ethylen und Buten-1 erhalten wird. Hierzu wird das in die Reaktion eingesetzte Ethylen zu 10 bis 80 % seiner Anfangsmenge, bevorzugt zu 25 bis 70 %, besonders bevorzugt zu 40 bis 60 %, umgesetzt.

Bei der Gewinnung eines solchen Gemisches von Ethylen und Buten-1 aus dem Reaktionsprodukt kann neben einer destillativen Abtrennung der beiden gewünschten Ge-

EC 144

mischbestandteile in besonders vorteilhafter Weise ein Abflashen dieser beiden Stoffe über eine geheizte Vorlage aus dem Reaktionsgemisch erfolgen. Bei diskontinuierlicher Durchführung des erfindungsgemäßen Verfahrens genügt beispielsweise anstelle eines solchen Abflashens die Gewinnung der beiden gewünschten Gemischbestandteile durch bloßes Entspannen des Reaktionsautoklaven. Das Buten-1 bzw. das Gemisch aus Ethylen und Buten-1 wird jeweils in einer entsprechend gekühlten Vorlage aufgefangen.

Für den Fall der Gewinnung eines solchen beschriebenen Ethylen/Buten-1-Gemisches hat dieses erfindungsgemäß hergestellte Gemisch ohne weitere Vorbehandlung eine für die Copolymerisation geeignete Reinheit (Polymerisation Grade). Ein solches Gemisch kann daher direkt in die Copolymerisation, beispielsweise mit Ziegler-Katalysatoren, eingesetzt werden. Es entfällt somit die sonst notwendige Vorbehandlung des Comonomeren Buten-1, das aus $C_4$-Schnitten fraktioniert wird. Solche Vorbehandlung, die aufgrund des erfindungsgemäßen Verfahrens vermieden werden kann, umfaßt beispielsweise die folgenden Schritte: Selektivhydrierung des $C_4$-Schnittes, Wasserwäsche zur Entfernung von Alkanolen und Carbonyl-Verbindungen und anschließende Trocknung, beispielsweise durch Molekularsiebe.

Die Vorteile des erfindungsgemäßen Verfahrens liegen in der Benutzung einfacher Katalysator-Komponenten, in der hohen Dimerisationsaktivität, in der hohen Selektivität zum Buten-1 und damit für den Fall der Herstellung eines Ethylen/Buten-1-Gemisches in der vereinfachten Prozeßführung für die Vorbereitung der Copolymerisation, da das beschriebene Gemisch in hoher Reinheit (Polymerisation Grade) anfällt und damit Reinigungsoperationen

EC 144

wie die Superfraktionierung der Butene oder die Entfernung von sauerstoffhaltigen Verbindungen, wie oben erwähnt, eingespart werden.

EC 144

Beispiele 1 bis 10

In einem stopfbuchslosen 3 l V4A-Büchi SFS-Rührautoklaven
wurden unter Sauerstoff- und Feuchtigkeitsausschluß die
in der Tabelle aufgeführten Mengen an Lösungsmitteln, Katalysator und Ethylen eingefüllt.

Das Reaktionsgemisch wurde unter Rühren schnell auf die
Reaktionstemperatur und den zugehörigen Reaktionsdruck
gebracht. Nach 90 Minuten wurde die Zusammensetzung des
ausreagierten Ansatzes gaschromatographisch untersucht.

EC 144

Tabelle    Beispiele 1 - 10

Lösungsmittel n-Hexan    500 ml, Reaktionszeit 90 Minuten

| Beispiel Nr. | Ethylen-Einsatz (g) | Übergangsmetall Bezeichnung | $\frac{mol}{mol}$ $C_2^=$ | Al-Komponente Bezeichn. | $\frac{mol}{mol}$ $C_2^=$ | Reakt.-Temperatur (°C) | Reakt.-Druck (bar) | Reaktionsgemisch ohne Lösungsmittel $C_2^=$ (g) | $C_4^=-1$ (g) | $C_4^=-2$ (g) | Rest-KW (g) | Ethylen-Umsatz (%) | Buten-1 Selektivität bez. auf n-Butene (%) |
|---|---|---|---|---|---|---|---|---|---|---|---|---|---|
| 1 | 33,0 | $(n-C_3H_7O)_4Ti$ | $10^{-3}$ | TIBA | $10^{-2}$ | 100 | 8,0 | 19,30 | 13,50 | 0,18 | 2,00 | 41,4 | 98,7 |
| 2 | 33,0 | $(i-C_3H_7O)_4Ti$ | $10^{-3}$ | IPRA | $10^{-2}$ | 100 | 9,5 | 18,74 | 14,11 | 0,12 | 2,00 | 43,2 | 99,2 |
| 3 | 33,0 | $(i-C_4H_9O)_4Ti$ | $10^{-3}$ | IPRA | $10^{-2}$ | 100 | 9,0 | 18,90 | 13,92 | 0,13 | 2,00 | 42,7 | 99,1 |
| 4 | 55,0 | $(n-C_3H_7O)_4Ti$ | $5 \cdot 10^{-4}$ | TIBA | $10^{-2}$ | 105 | 15,0 | 29,54 | 25,26 | 0,20 | 3,30 | 46,3 | 99,2 |
| 5 | 55,0 | $(i-C_3H_7O)_4Ti$ | $10^{-4}$ | TIBA | $5 \cdot 10^{-3}$ | 105 | 15,0 | 30,03 | 24,85 | 0,10 | 1,67 | 45,4 | 99,6 |
| 6 | 55,0 | $(n-C_3H_7O)_4Zr$ | $5 \cdot 10^{-4}$ | TIBA | $10^{-2}$ | 108 | 15,0 | 45,49 | 9,34 | 0,14 | 3,30 | 17,3 | 98,5 |
| 7 | 55,0 | $(i-C_3H_7O)_4Ti$ | $2,5 \cdot 10^{-4}$ | TIBA | $5 \cdot 10^{-3}$ | 110 | 15,0 | 26,42 | 28,25 | 0,10 | 1,88 | 52,0 | 99,6 |
| 8 | 55,0 | $(n-C_4H_9O)_4Ti$ | $2,5 \cdot 10^{-4}$ | TIBA | $5 \cdot 10^{-3}$ | 109 | 15,0 | 28,22 | 26,43 | 0,15 | 1,85 | 48,7 | 99,4 |
| 9 | 84,0 | $(i-C_3H_7O)_4Ti$ | $5 \cdot 10^{-4}$ | IPRA | $5 \cdot 10^{-3}$ | 108 | 30,0 | 43,09 | 39,68 | 1,23 | 0,89 | 48,7 | 97,0 |
| 10 | 84,0 | $(i-C_3H_7O)_4Ti$ | $5 \cdot 10^{-4}$ | TIBA | $5 \cdot 10^{-3}$ | 105 | 33,0 | 45,70 | 37,83 | 0,44 | 0,87 | 45,6 | 98,9 |

TIBA = Triisobutylaluminium

IPRA = Isoprenylaluminium

$C_2^=$ = Ethylen

$C_4^=-1$ = Buten-1

$C_4^=-2$ = Buten-2

Beispiel 11

Die Gasphase aus dem Dimerisierungsautoklaven nach Versuch 8 wurde über eine Stahlkapillare, in einen 1,5 l Büchi SFS-Rührautoklaven entspannt und dort in einer Fällungspolymerisation direkt polymerisiert.

Die Zusammensetzung des übergeführten Ethylen/Buten-1-Gemisches wurde gaschromatographisch analysiert.

| Die überführte Gasmenge betrug: | | 29,2 Normliter |
| Die Zusammensetzung war: | Ethylen | 71,1 Vol.-% |
| | Buten-1 | 26,8 Vol.-% |
| | Buten-2 | 0,2 Vol.-% |
| | Rest-KW | 1,9 Vol.-% |

Die Polymerisation wurde in 600 ml Toluol bei 40 bis 50°C und 2,6 bar über 30 Minuten durchgeführt.

Als Katalysatorsystem wurde $3 \times 10^{-4}$ Mol $TiCl_4$
$\phantom{Als Katalysatorsystem wurde} + \phantom{3 \times} 10^{-4}$ Mol $VOCl_3$
$\phantom{Als Katalysatorsystem wurde} + \phantom{3 \times} 10^{-4}$ Mol TIBA
verwendet.

Nach Abschluß der Polymerisation wurde der Glasautoklav entspannt, das Restgas 10,6 Normliter gaschromatographisch untersucht.

| Die Zusammensetzung war: | Ethylen | 40,1 Vol.-% |
| | Buten-1 | 55,3 Vol.-% |
| | Buten-2 | 0,5 Vol.-% |
| | Rest-KW | 4,1 Vol.-% |

Die Ausbeute an LLDPE betrug 21,7 g, die Dichte des Produktes war 0,9250.

EC 144

## Patentansprüche

1) Verfahren zur Herstellung von Buten-1 durch Dimerisierung von Ethylen in Gegenwart eines Mischkatalysators aus einem Titan- oder Zirkonsäurealkylester und einem Aluminiumorganyl bei erhöhter Temperatur in einem Lösungsmittel, dadurch gekennzeichnet, daß man zwischen 90 und 200°C arbeitet und $1 \times 10^{-6}$ bis $5 \times 10^{-3}$ Mol der Titan- oder Zirkonkomponente und $1 \times 10^{-4}$ bis $3 \times 10^{-2}$ Mol der Aluminiumkomponente pro Mol Ethylen einsetzt.

2) Verfahren nach Anspruch 1, dadurch gekennzeichnet, daß man 92 bis 150°C arbeitet.

3) Verfahren nach Anspruch 1, dadurch gekennzeichnet, daß man bei 95 bis 130°C arbeitet.

4) Verfahren nach Ansprüchen 1 bis 3, dadurch gekennzeichnet, daß man $1 \times 10^{-5}$ bis $1 \times 10^{-3}$ Mol der Titan- oder Zirkonkomponente einsetzt.

5) Verfahren nach Ansprüchen 1 bis 4, dadurch gekennzeichnet, daß man $5 \times 10^{-4}$ bis $1 \times 10^{-2}$ Mol der Aluminiumkomponente einsetzt.

6) Verfahren nach Ansprüchen 1 bis 5, dadurch gekennzeichnet, daß man bei 5 bis 50 bar Gesamtdruck des Reaktionssystems arbeitet.

EC 144

7) Verfahren zur Herstellung eines Gemisches aus Ethylen und Buten-1 nach Ansprüchen 1 bis 6, dadurch gekennzeichnet, daß man das Ethylen zu 10 bis 80 % seiner Anfangsmenge umsetzt.

8) Verfahren nach Anspruch 7, dadurch gekennzeichnet, daß man das Ethylen zu 25 bis 70 % umsetzt.

9) Verfahren nach Ansprüchen 7 und 8, dadurch gekennzeichnet, daß man das Ethylen/Buten-1-Gemisch durch Abflashen aus dem Reaktionsgemisch gewinnt.

10) Verwendung von Ethylen/Buten-1-Gemischen nach Anspruch 7 zur direkten Herstellung von Ethylen/Buten-1-Copolymerisaten.

EC 144

Europäisches
Patentamt

**EUROPÄISCHER RECHERCHENBERICHT**

**0114416**
Nummer der Anmeldung

EP 83 11 3257

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. ³) |
|---|---|---|---|
| A | DE-B-1 018 857 (K. ZIEGLER) <br> * Patentansprüche * <br><br> ----- | 1-3,6 | C 07 C 11/08 <br> C 07 C 2/30 <br> C 08 F 210/02 // <br> (C 08 F 210/02 <br> C 08 F 210/08 ) |

RECHERCHIERTE
SACHGEBIETE (Int. Cl. ³)

C 07 C 11/00
C 07 C 2/00

Der vorliegende Recherchenbericht wurde für alle Patentansprüche erstellt.

| Recherchenort <br> DEN HAAG | Abschlußdatum der Recherche <br> 19-03-1984 | Prüfer <br> VAN GEYT J.J.A. |
|---|---|---|